Europäisches Patentamt

European Patent Office

Office européen des brevets

(11) Publication number: **0 136 392**
**B1**

# EUROPEAN PATENT SPECIFICATION

(45) Date of publication of patent specification: **31.05.89**

(21) Application number: **84100644.8**

(22) Date of filing: **21.01.84**

(51) Int. Cl.⁴: **A 61 M 5/32**

(54) **Mini electrical syringe needle destroyer.**

(30) Priority: **08.08.83 JP 143934/83**

(43) Date of publication of application:
**10.04.85 Bulletin 85/15**

(45) Publication of the grant of the patent:
**31.05.89 Bulletin 89/22**

(84) Designated Contracting States:
**AT BE CH DE FR GB IT LI LU NL SE**

(56) References cited:
**WO-A-79/00239**
**WO-A-82/00412**
**US-A-4 255 996**
**US-A-4 375 849**

(73) Proprietor: **Hsieh, Ch'ing-Lung**
**113,5th F.,Sect.5 Yen-Ping North Road**
**Taipei City (TW)**

(72) Inventor: **Hsieh, Ch'ing-Lung**
**113,5th F.,Sect.5 Yen-Ping North Road**
**Taipei City (TW)**

(74) Representative: **Meyer, Ludgerus**
**Patentanwälte Meyer, Stach, Vonnemann**
**Jungfernstieg 38**
**D-2000 Hamburg 36 (DE)**

Courier Press, Leamington Spa, England.

## Description

The invention relates to a device for destroying and removing syringe needles, i.e. cannulae, from syringe barrels.

A great concern to hospital staff personnel, particularly medical technicians, is quick and safe disposal of needles after injection or blood sampling. When manual disposition is required the chance for skin puncturing and scratching from the sharp end of a cannular needle occurs all too often. In fact, needle accidents sustained in this manner are among the most frequent causes of injury to hospital workers. Such injuries also are the source of potential infections to the hospital personnel. It would accordingly be desirable for medical personnel to be able to dispose of these items safely and easily.

A wide assortment of devices has been provided through the years to make disposal techniques safe and fast. Many involve mere needle removal from a syringe body and others involve the preferred practice of needle severance to ensure that no re-use occurs. Yet another variety destroys the syringe as well as the needle for particular application with hypodermic syringes used for injections.

One previous solution affords removal of friction-fit needles by the insertion of the needle through an opening into a collection box. The head, or hub, of the needle is caught behind a cover plate and pulling on the syringe body detaches the needle.

US Patent 4,375,849 discloses a similar but improved syringe needle removal and disposal device.

US Patent No 3,914,865 describes a device that cuts both the metal cannula and attaches plastic hub or syringe barrel with separately actuated cutters. Another device marketed by Becton Dickinson under the trade mark DESTRUCLIP is a box-like device that cuts the cannula and syringe barrel or hub in separately actuated cutting steps. The reason these devices required two cutting motions was because the stainless steel cannula required a very high cutting force over a small distance, i.e., cannula diameter. Conversely, the much larger diameter thermoplastic hub or barrel required a greater distance of travel during its cutting step, but required less force because the thermoplastic material was softer than the metal cannula. This double cutting motion required by an operator was tedious in that there were two manual cutting motions required, as well as two insertions of the needle or syringe into the device.

Other syringe and needle destroyers such as in US Patent Nos 3,404,593 and 3,785,233, which made both cuts a simultaneous operator motion, were cumbersome. They required very large levers to get both the long distance cutter travel for severing the large diameter hub or syringe barrel and also get the required cutting force for the metal cannula. Such units usually require the full arm motion of the operator.

A similar device is disclosed in US Patent 4,255,996 where many of the above problems are overcome by providing hub and cannula cutters that are joined at a slidable joint and pivoted at noncoaxial pivot points to provide increased mechanical advantage to the cannula cutter. In one preferred embodiment, the same type of cutting mechanism has its cutters spaced so as to sever a hypodermic syringe barrel and a cannula of a needle attached to the syringe barrel in a single motion.

According to the invention, there is provided a syringe needle destruction device as defined in claim 1. Particular embodiments of the invention are set out in the dependant claims.

As will be seen from the illustrative drawings which follow, the invention provides, in preferred forms, an electrical syringe needle destroyer with a needle or cannula detaching device which comprises a round needle head-receiving hole located on top of the outer case, i.e., overlying two noncontacting electrodes of the needle destroying device, and a needle detaching slide track, one end of the track connecting to the needle head-receiving hole, of which the width is between the neck of the injector and the shoulder of the needle-head.

A major advantage of the device of the invention is that the user simply holds the syringe barrel and introduces the used cannula into the device so that the cannula is in contact with both electrodes. At this moment, the used cannula will absorb the strong current of the resulting circuit between the two electrodes.

A further advantage of the invention in its preferred embodiments is that the destruction, the detachment, and the collection of the needles can be done in one combined operation, thus simplifying use.

The following specific description of a preferred embodiment is intended to illustrate the invention, by way of example only, reference being made to the accompanying drawings in which:—

Figure 1 is a perspective view of a syringe needle destruction device according to the invention, with internal detail shown and the outer casing represented in broken lines;

Figure 2 is a view, similar to Figure 1, but showing the device rotated through 180°, the exterior surface of the casing being shown except for a small portion broken away to expose internal detail;

Figure 3 is a cross-section through the portion of the device encircled in Figure 2, additionally showing a syringe and needle assembly in position for needle destruction;

Figure 4 shows part of the syringe needle destruction device as a cross section taken along the line A—A of Figure 2;

Figure 5 shows the position of a syringe and needle assembly in relation to the syringe needle destruction device at various of the stages involved in needle destruction; and

Figure 6 shows, on an enlarged scale, the second and third stages represented in Figure 5 in terms of the more detailed relationship between the syringe and needle assembly and the destruction device.

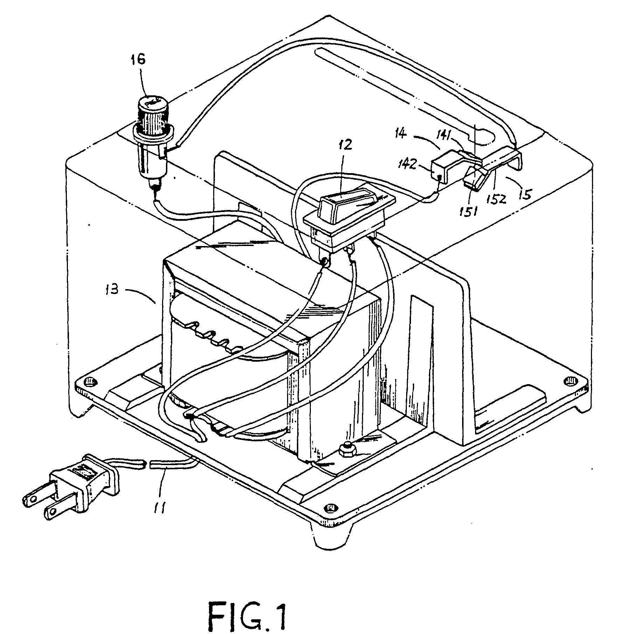

The syringe needle destruction device shown in the Figures comprises an outer casing 2 permanently housing various of the components hereinafter referred to and a collection box 22 which, as will be appreciated from the Figures, functions as a drawer which can be withdrawn from and reintroduced into the cavity of the casing 2 by a sliding action. An electric power lead 11 (Figure 1) terminates at one extremity in a 2-pin connection plug intended in the embodiment shown for coupling to a 110 volt or 220 volt power supply as normally available in a typical use environment. The other end of lead 11 connects to a terminal of an electrical switch 12, two connecting wires respectively connecting two further terminals of the switch to a transformer 13. Electrode 14 of a pair of electrodes 14, 15 is connected to the output of the transformer 13 by means of a further wire, a still further wire connecting to electrode 15 thereby bringing fuse 16 into the circuit for protection purposes.

Step-down transformer 13 delivers to electrodes 14 and 15 a voltage of no more than 3 to 4 volts thus limiting the fuse current to a safe level in the range 5 to 30 amps.

Electrode 14 comprises a portion 142 to which electrical power connection is made from the transformer output, this portion comprising gold-plated copper alloy. A further portion 141 defines an electrical contact surface and is made of platinum-coated high melting point metallic material. Electrode 15 is similarly constructed with a portion 152 made of gold-plated copper alloy and an electrical contact portion 151 made of platinum-coated high melting point metallic material.

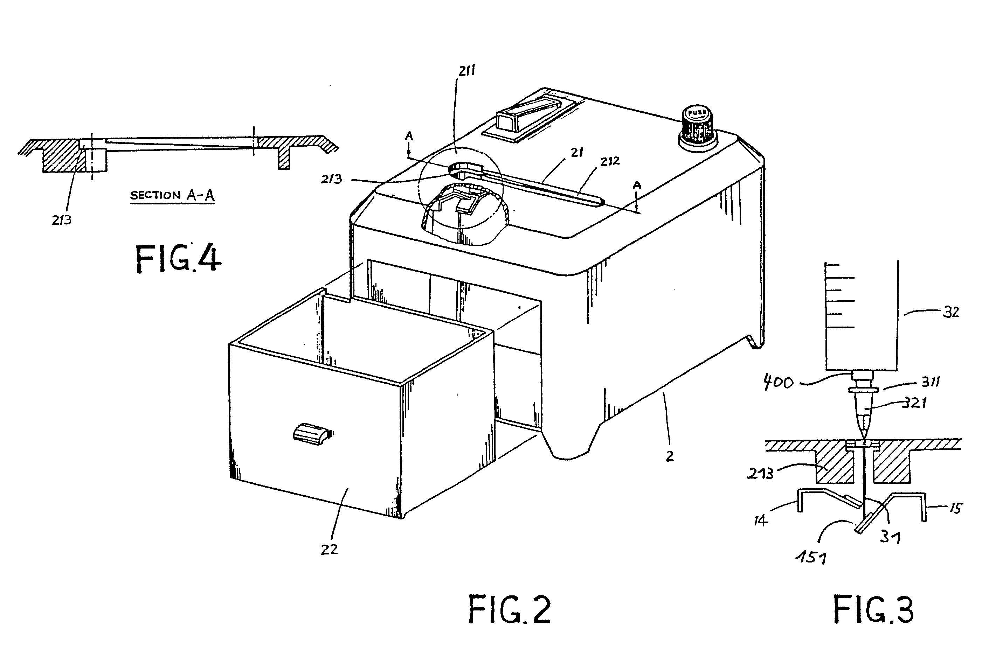

The upwardly facing surface of the casing 2 is provided with a cannula-removal assembly 21 (Figure 2). Assembly 21 comprises a cannula-receiving orifice 211 and an elongate slot 212 both penetrating into the cavity of the casing 2, electrodes 14, 15 being disposed immediately beneath orifice 211 with electrical contact surfaces 141, 151 essentially in register therewith.

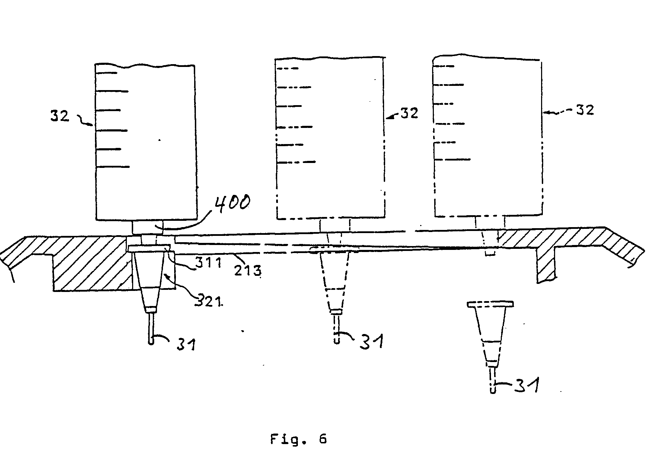

A support frame 213 equivalent in width to that of slot, 212 is provided beneath slot 212. Frame 213 and the margins of the casing 2 about slot 212 define a gap which decreases in depth with spacing from orifice 211, as best illustrated in Figure 6 of the drawings.

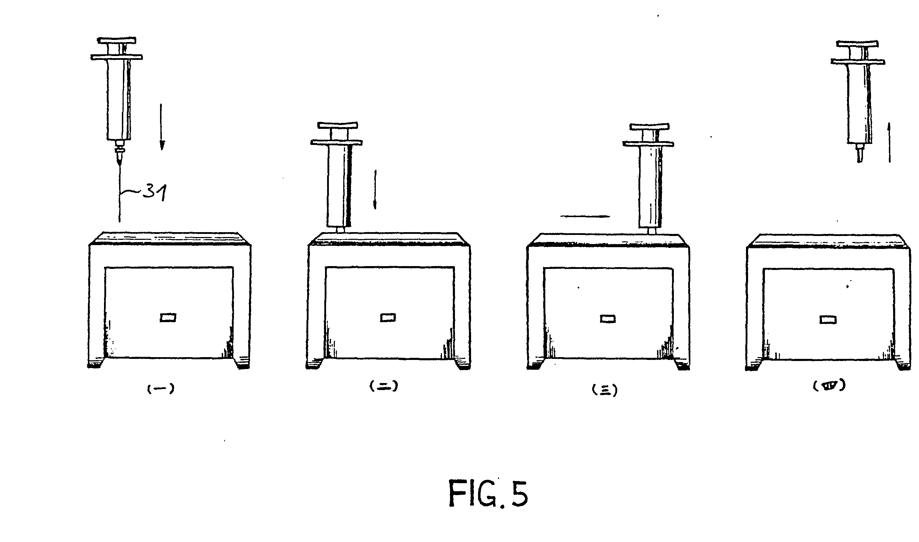

In use, the device shown in the Figures is first energized by operation of switch 12. A cannula 31 fixed to a syringe barrel 32 is then introduced into orifice 211 (Figures 3 and 5). The cannula 31 is extended into the cavity of casing 2 until the needle tip makes contact with the electrical contact surface 151 of electrode 15. At this point, as best illustrated in Figure 3, the needle also makes contact by its circumference with the electrical contact surface 141 of electrode 14 to produce an electrical current across the electrodes whilst hub 321 and the syringe reservoir remain external of the casing.

The cross-sectional area of the cannula 31 is very small and accordingly its electrical resistance is larger than that of electrodes 14 and 15. The electrical current within the circuit therefore melts the material of the cannula thus causing the cannula to undergo deformation. This in turn enables further depression of the syringe assembly as a whole into the cavity of the casing 2, limited only by ultimate abutment of shoulder 311 of the hub 321 upon the gap-defining surface of support frame 213.

At this point, the syringe assembly as a whole is displaced laterally from orifice 211 into and along slot 212. Upon entry into slot 212, the marginal portions of the casing 2 are sandwiched between hub shoulder 321 and a shoulder 400 represented by the neck of the barrel 32 of the syringe assembly (Figures 3 and 6). Further such lateral displacement of the syringe assembly urges hub shoulder 321 and shoulder 400 apart until, as best seen in Figure 6 of the drawings, the cannula 31 has been completely separated from the syringe barrel 32. Upon separation, the now deformed cannula descends into the cavity of collection box 22, a heat-insulating metal plate being provided in box 22 for receipt thereof.

## Claims

1. A syringe needle destruction device for application to syringe assemblies in which a syringe barrel (32) is equipped with an electrically conductive cannula (31), which device comprises a housing (2), cannula storage means (22), cannula destruction means (14, 15) and separation means (21) provided by said housing (2) for separating the syringe cannula (31) from the syringe barrel (32) whereby the separated cannula (31) is collected in the cannula storage means (22), characterized in that said cannula destruction means (14, 15) comprises electrical means (11, 13, 14, 15) for passing an electrical current surge through said cannula (31) to melt the material thereof and deform the cannula (31).

2. A device as claimed in Claim 1 wherein the cannula storge means (22) comprises a collection box disposed within said housing (2).

3. A device as claimed in Claim 1 or Claim 2 wherein the cannula destruction means (14, 15) comprises two spaced apart electrodes (14, 15) arranged that the cannula (32) completes an electrical circuit including a transformer (13) and a fuse (16) when introduced between the electrodes (14, 15).

4. A device as claimed in any preceding claim wherein the housing (2) has an opening (211, 212) penetrating a housing (2) wall and including a cannula-receiving orifice (211), an adjacent slot (212) communicating therewith and a support frame (213) beneath said slot (212) for supporting a hub (311) of the cannula (31), said frame (213) sandwiching the portions of the housing wall marginal to the slot (212), said portions being arranged to come into co-operation with a shoulder (400) of the syringe barrel (32) and to form together with said support frame (213), wedge-shaped edges along the slot (212) so that as the syringe assembly is displaced along the slot (212) from said orifice (211), the cannula (31) and the barrel (32) are urged apart and separated from each other.

## Patentansprüche

1. Einrichtung zur Zerstörung von Spritzennadeln von zusammengesetzten Spritzen, bei denen ein Spritzenbehälter (32) mit einer elektrisch leitenden Kanüle (31) vesehen ist, wobei die Einrichtung aufweist: ein Gehäuse (2), einen Kanülenaufnahmebehälter (22), eine Kanülenzerstörungsanordnung (14, 15) und Trennmittel (21), die am Gehäuse (2) vorgesehen sind, um eine Spritzenkanüle (31) vom Spritzenbehälter (32) abzutrennen, wobei die abgetrennte Kanüle (31) im Kanülenaufnahmebehälter (22) gesammelt wird, dadurch gekennzeichnet, daß die Kanülenzerstörungseinrichtung (14, 15) elektrische Mittel (11, 13, 14, 15) zur Durchleitung eines elektrischen Stromstoßes durch die Kanüle (31) aufweist, um dessen Material zu schmelzen und die Kanüle (31) zu verformen.

2. Einrichtung nach Anspruch 1, bei der der Kanülenaufnahmebehälter (22) eine innerhalb des Gehäuses (2) angeordnete Sammelbox enthält.

3. Einrichtung nach Anspruch 1 oder 2, bei der die Kanülenzerstörungsanordnung (14, 15) zwei mit Abstand angeordnete Elektroden (14, 15) enthält, die so angeordnet sind, daß die Kanüle einen elektrischen Stromkreis mit einem Transformator (13) und einer Sicherung (16) bildet, wenn die Kanüle zwischen die Elektroden (14, 15) eingeführt ist.

4. Einrichtung nach einem der vorhergehenden Ansprüche, bei der das Gehäuse (2) eine Öffnung (211, 212) in einer Gehäusewand aufweist, die eine Kanülen-Einführungs-Öffnung (211) enthält, an die ein Spalt (212) anschließt, und ein Führungsrahmen (213) unterhalb des Spaltes (212) zur Führung eines Flansches (311) der Kanüle (31) vorgesehen ist, wobei der Rahmen (213) an den Teilen des Gehäuses um den Spalt herum angeordnet ist, bei dem die Teile mit einer Schulter (400) des Spritzenbehälters (32) in Verbindung treten, und zusammen mit dem Führungsrahmen (213) keilförmige Kanten entlang des Spaltes (212) bilden, so daß bei Verschieben der zusammengesetzten Spritze entlang des Spaltes (212) von der Öffnung (211) weg die Kanüle (31) und der Behälter (32) auseinandergedrückt und voneinander getrennt werden.

## Revendications

1. Appareil pour la destruction d'aiguilles de seringues, destiné à des ensembles formant seringues comprenant un corps de seringue (32) mini d'une canule (31) conductrice d'électricité, lequel appareil comporte un boîtier (2), des moyens de stockage de canules (22), des moyens de destruction de canules (14, 15), et des moyens de séparation de canules (21) définis par ledit boîtier (2), en vue de séparer la canule de seringue (31) du corps de seringue (32), la canule (31) séparée étant recueillie dans les moyens de stockage de canules (22), et est caractérisé en ce que lesdits moyens de destruction de canules (14, 15) comportent des moyens électriques (11, 13, 14, 15) destinés à faire passer une impulsion de courant électrique à travers ladite canule (31) pour faire fondre le matériau de celle-ci et la déformer.

2. Appareil tel qui défini dans la revendication 1, dans lequel les moyens de stockage de canules (22) comportant une boîte collectrice disposée à l'intérieur dudit boîtier (2).

3. Appareil tel que défini dans la revendication 1 ou la revendication 2, dans lequel les moyens de destruction de canules (14, 15) comportent deux électrodes (14, 15) espacées l'une de l'autre et conçues pour que la canule (31) établisse, lors de son insertion entre celles-ci, un circuit électrique comportant un transformateur (13) et un fusible (16).

4. Appareil tel que défini dans l'une quelconque des revendications précédentes, dans lequel le boîtier (2) présente une ouverture (211, 212) traversant l'une de ses parois et comportant un orifice de réception de canules (211), un fente adjacente (212) communiquant avec ce dernier, et une structure de support (213) placée sous ladite fente (212), en vue de supporter une embase (311) de la canule (31), ladite structure (213) prenant en sandwich les parties de la paroi de boîtier qui bordent le fente (212), tandis que lesdites parties sont conçues pour venir coopérer avec un épaulement (400) du corps de seringue (32) et pour, conjointement avec ladite structure (213), définer le long de la fente (212) des arêtes en forme de coins, si bien que, lorsque l'ensemble formant seringue est déplacé le long de la fente (212) en partant dudit orifice (211), la canule (31) et le corps (32) sont sollicités pour s'écarter réciproquement et sont séparés l'un de l'autre.

FIG.1

SECTION A-A

213

FIG.4

211

A

213

21 212

A

PUSH

2

22

EP 0 136 392 B1

400

311

321

32

213

14

151

31

15

FIG.2

FIG.3

FIG.5

Fig. 6